# EUROPÄISCHE PATENTSCHRIFT

(11) **EP 0 682 051 B1**
(45) Veröffentlichungstag und Bekanntmachung des Hinweises auf die Patenterteilung: **10.11.1999**
(21) Anmeldenummer: 95106601.8
(22) Anmeldetag: 01.05.1995
(51) Int. Cl.: C08G 18/80

(54) **Mischblockierte Polyisocyanate**
Polyisocyanates blocked by a mixture of blocking agents
Polyisocyanates bloqués par un mélange d'agents de blocage

(30) Priorität: 13.05.1994 DE 4416750
(43) Veröffentlichungstag der Anmeldung: 15.11.1995
(73) Patentinhaber: BAYER AG, 51368 Leverkusen (DE)
(72) Erfinder: König, Eberhard, Dr., D-51375 Leverkusen (DE); Engbert, Theodor, Dr., D-50968 Köln (DE)

(56) Entgegenhaltungen:
- EP-A- 0 500 495
- EP-A- 0 600 314
- US-A- 3 248 398
- US-A- 3 721 645

## Beschreibung

Die Erfindung betrifft neue blockierte Polyisocyanate, die die Herstellung von, bei vergleichsweise niedrigen Temperaturen von 120 - 140 °C einbrennbaren, Einkomponenten-Polyurethanlacken mit wesentlich erniedrigtem Thermogilb gestatten, ein Verfahren zu ihrer Herstellung und ihre Verwendung.

Mehrschichtlackierungen, die als Decklack einen glänzenden Klarlack auf Basis von blockierten Polyisocyanaten und organischen Polyhydroxylverbindungen, beispielsweise Polyhydroxypolyacrylaten aufweisen, gewinnen wegen ihrer ausgezeichneten lacktechnischen Eigenschaften, insbesondere bei der Automobillackierung, immer größere Bedeutung.

Für dieses spezielle Einsatzgebiet geeignete blockierte Polyisocyanate müssen insbesondere (i) bei einer Einbrenntemperatur von max. 140°C vernetzbar sein, (ii) während des Einbrennvorgangs allenfalls eine sehr geringe, vorzugsweise überhaupt keine Thermovergilbung aufweisen, (iii) in konzentrierter organischer Lösung eine für "high solid"-Anwendungen ausreichend niedrige Viskosität aufweisen und (iv) auf preisgünstigen Rohstoffen basieren.

Die 1,2,4-Triazol-blockierten Polyisocyanate gemäß EP-B 0 004 571, die insbesondere als Pulverlack-Vernetzer in Betracht gezogen werden, sind für einen Einsatz in Lösungsmittel enthaltenden Lacken weitgehend ungeeignet, da ihre Lösungen in organischen Lösungsmitteln vergleichsweise hochviskos und oftmals wegen der Kristallisationsneigung der blockierten Polyisocyanate instabil sind.

Jetzt wurde überraschend gefunden, daß die vorstehend genannten Bedingungen in optimaler Weise erfüllt werden, wenn zur Blockierung der üblichen Lackpolyisocyanate anstelle von reinem 1,2,4-Triazol eine Kombination dieses Blockierungsmittels mit 3,5-Dimethylpyrazol, sowie gegebenenfalls weiteren an sich bekannten Blockierungsmitteln zum Einsatz gelangt.

Sowohl 1,2,4-Triazol als auch 3,5-Dimethylpyrazol sind im Prinzip bekannte Blockierungsmittel für Isocyanatgruppen, die beide in US-PS 3 248 398 als Blockierungsmittel für langkettige aliphatische Monoisocyanate erwähnt werden. 3,5-Dimethylpyrazol wird außerdem in der EP-A-0 159 117 neben anderen Pyrazolen als Blockierungsmittel für organische Polyisocyanate beschrieben. Die blockierten Polyisocyanate sollen insbesondere als Komponente für Elektrotauchlacke eingesetzt werden, die in der Regel überlackiert werden, so daß es auf die Thermostabilität dieser Grundierungen nicht ankommt. In der Vorveröffentlichung findet sich demzufolge auch keinerlei Hinweis auf die Eignung dieses Blockierungsmittels zur Herstellung von blockierten Polyisocyanaten einer hohen Thermostabilität.

Gegenstand der Erfindung sind (cyclo)aliphatische Polyisocyanate, deren Isocyanatgruppen zu mindestens 95 % in mit Blockierungsmitteln blockierter Form vorliegen, und die einen Gehalt an unblockierten und blockierten Isocyanatgruppen (berechnet als NCO) von insgesamt 5 bis 25 Gew.-% aufweisen, dadurch gekennzeichnet, daß sich die Blockierungsmittel zu
A) 30 bis 70 Äquivalent-% aus 1,2,4-Triazol, zu
B) 30 bis 70 Äquivalent-% aus 3,5-Dimethylpyrazol und zu
C) 0 bis 30 Äquivalent-% aus anderen, von A) und B) verschiedenen Blockierungsmitteln zusammensetzen,
wobei sich die genannten Prozentsätze auf die Gesamtheit der Blockierungsmittel beziehen und zu 100 ergänzen.

Gegenstand der Erfindung ist auch ein Verfahren zur Herstellung dieser Polyisocyanate, gegebenenfalls in in Lacklösungsmitteln gelöster Form, durch Umsetzung einer, gegebenenfalls in Lacklösungsmitteln gelösten Polyisocyanat-Komponente, bestehend aus mindestens einem Polyisocyanat mit (cyclo)aliphatisch gebundenen Isocyanatgruppen und einem Isocyanatgehalt von 7 bis 30 Gew.-% mit Blockierungsmitteln für Isocyanatgruppen, wobei gegebenenfalls vor, während und/oder im Anschluß an die Blockierungsreaktion bis zu 20 NCO-Äquivalent-% des zu blockierenden Polyisocyanats mit Verbindungen mit mindestens einer Carbonsäurehydrazid-Gruppe und mindestens einer gegenüber Isocyanatgruppen reaktionsfähigen Gruppe im Sinne einer Isocyanat-Additionsreaktion zur Reaktion gebracht werden, so daß das resultierende, blockierte Polyisocyanat bis zu 5 Gew.-%, bezogen auf Feststoff, an chemisch fixierten Struktureinheiten der Formel 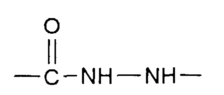 enthält,
dadurch gekennzeichnet, daß die Blockierungsmittel in einer Menge von mindestens 95 Äquivalent-%, bezogen auf die, nach der genannten, gegebenenfalls durchzuführenden Modifizierung noch vorliegenden NCO-Gruppen des zu blockierenden Polyisocyanats zum Einsatz gelangen und sich, bezogen auf die Gesamtmenge der Blockierungsmittel, zu
A) 30 bis 70 Äquivalent-% aus 1,2,4-Triazol, zu
B) 30 bis 70 Äquivalent-% 3,5-Dimethylpyrazol und zu
C) 0 bis 30 Äquivalent-% aus mindestens einem weiteren, von A) und B) verschiedenen Blockierungsmittel zusammensetzen, wobei die Blockierungsmittel in beliebiger Reihenfolge oder als Gemisch zum Einsatz gelangen.

Gegenstand der Erfindung ist schließlich auch die Verwendung der erfindungsgemäßen blockierten Polyisocyanate als Vernetzer für organische Polyhydroxylverbindungen in Polyurethan-Einkomponenten-Einbrennlacken.

Bei den den erfindungsgemäßen blockierten Polyisocyanaten zugrundeliegenden Polyisocyanaten handelt es sich um an sich bekannte Lackpolyisocyanate mit aliphatisch und/oder cycloaliphatisch gebundenen Isocyanatgruppen und einem Isocyanatgehalt von 7 bis 30, vorzugsweise 12 bis 25 Gew.-%. In Betracht kommen insbesondere die an sich bekannten, Biuret-, Isocyanurat- und/oder Uretdiongruppen aufweisenden Lackpolyisocyanate auf Basis von 1,6-Diisocyanatohexan (HDI), 1-Isocyanato-3,3,5-trimethyl-5-isocyanatomethyl-cyclohexan (IPDI) und/oder 4,4'-Diisocyanato-dicyclohexylmethan. Besonders bevorzugt sind Isocyanuratgruppen aufweisende Lackpolyisocyanate auf Basis von (i) IPDI, (ii) Gemischen aus IPDI und 4,4'-Diisocyanatodicyclohexylmethan oder (iii) 1,6-Diisocyanatohexan.

Als Blockierungsmittel A) wird 1,2,4-Triazol, als Blockierungsmittel B) 3,5-Dimethylpyrazol eingesetzt. 1,2,4-Triazol ist hierbei ein im Markt befindliches Handelsprodukt. 3,5-Dimethylpyrazol kann beispielsweise durch Kondensation von äquimolaren Mengen von Acetylaceton und Hydrazinhydrat und Auskreisen des Reaktions- und Hydratwassers mittels Toluol erhalten werden. Als weitere, gegebenenfalls mitzuverwendende Blockierungsmittel C) kommen von den Blockierungsmitteln A) und B) verschiedene Blockierungsmittel wie z. B. Oxime wie Butanonoxim, sekundäre Amine wie Diisopropylamin oder Imidazol, CH-acide Verbindungen, z. B. Malonsäurediethylester oder ε-Caprolactam in Betracht.

Gemäß einer bevorzugten Ausführungsform des erfindungsgemäßen Verfahrens werden die zu blockierenden Ausgangspolyisocyanate vor, während und/oder im Anschluß an die Blockierungsreaktion mit bis zu 20, vorzugsweise 1 bis 12 Äquivalent-% (bezogen auf die Isocyanatgruppen der Ausgangspolyisocyanate einerseits und die gegenüber Isocyanatgruppen reaktionsfähigen Gruppen der Hydrazin-Addukte andererseits) an Verbindungen umgesetzt, die pro Molekül mindestens eine Struktureinheit der Formel 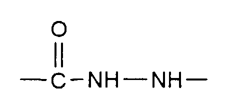 und mindestens eine, von der genannten Struktureinheit verschiedene, gegenüber Isocyanatgruppen reaktionsfähige Gruppe aufweisen. Im übrigen werden die Mengenverhältnisse der Reaktionspartner bei dieser Modifizierung vorzugsweise so gewählt, daß die erfindungsgemäßen blockierten Polyisocyanate bis zu 5 Gew.-% an Struktureinheiten der letztgenannten Formel enthalten.

Für diese Modifizierungsreaktion geeignete Hydrazin-Addukte sind beispielsweise solche der Formel 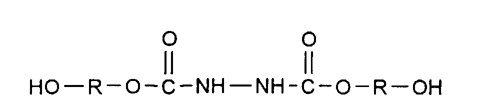 in welcher,
- R: für einen Ethylen-, Isopropylen- oder 1,3-Neopentylen-Rest
steht.

Die Hydrazin-Addukte können durch Umsetzung von 1 Mol Hydrazinhydrat mit 2 Mol eines cyclischen Carbonats, z. B. Ethylen-, Isopropylen- oder 1,3-Neopentylencarbonat, beispielsweise in siedendem Toluol erhalten werden. Hydrat- und Reaktionswasser werden hierbei azeotrop abdestilliert.

Andere, für die erfindungsgemäße Modifizierungsreaktion geeignete Hydrazin-Addukte sind beispielsweise die in US-PS 5 216 078 genannten Verbindungen, insbesondere das von der Firma Elf-Atochem unter der Bezeichnung ®Luchem HA-R 100 vertriebene Hydrazin-Addukt der Formel

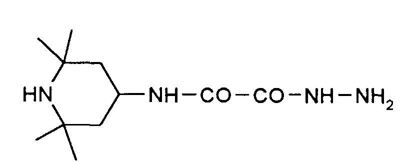

Die Blockierungsmittel A) bis C) werden bei der Durchführung des erfindungsgemäßen Verfahrens in solchen Mengen eingesetzt, daß das Äquivalentverhältnis von Isocyanatgruppen der Ausgangspolyisocyanate abzüglich der gegebenenfalls zum Einbau des Hydrazin-Adduktes benötigten NCO-Gruppen zu NCO-reaktiven Gruppen des Blockierungsmittels bei 0,95:1 bis 1,1:1 liegt. Die Isocyanatgruppen der erfindungsgemäßen Polyisocyanate sind demzufolge zumindest zu 95 %, vorzugsweise vollständig blockiert.

Die Durchführung des erfindungsgemäßen Verfahren erfolgt im allgemeinen im Temperaturbereich von 50 bis 120, vorzugsweise 60 bis 100 °C in Substanz oder in geeigneten Lösungsmitteln wie beispielsweise n-Butylacetat, Methoxypropylacetat, Toluol oder höheren aromatischen Lösungsmittelgemischen wie sie beispielsweise von der Firma Exxon-Chemie unter der Bezeichnung ®Solvesso vertrieben werden.

Beispielsweise kann das erfindungsgemäße Verfahren nach folgender Verfahrensvariante durchgeführt werden: Man legt die gelöste Isocyanatkomponente vor und gibt hierzu die erste Teilmenge des Blockierungsmittels, beispielsweise die Komponente A). Die Umsetzung mit 1,2,4-Triazol wird bei 100 °C in ca. 1 Stunde bis zu dem kalkulierten NCO-Gehalt durchgeführt. Anschließend kühlt man ab, z. B. auf 60 °C, und setzt mit der anderen Teilmenge der reaktionsfreudigeren Komponente B) um, vorzugsweise bis kein NCO-Gehalt IR-spektroskopisch mehr nachweisbar ist. Gegebenenfalls mit Lösungsmittel wird zum Schluß auf die gewünschte Viskosität eingestellt.

Im Falle einer zusätzlichen Modifizierung der erfindungsgemäßen blockierten Polyisocyanate durch Einbau von Hydrazin-Addukten der beispielhaft genannten Art können diese im Prinzip zu einem beliebigen Zeitpunkt vor, während und/oder im Anschluß an die Blockierungsreaktion eingebaut werden. So kann man beispielsweise bei Verwendung der obengenannten Hydroxylgruppen aufweisenden Hydrazin-Addukte diese nach Blockierung mit dem Blockierungsmittel A) und vor dem Einsatz des Blockierungsmittel B) dem Reaktionsgemisch hinzufügen.

Die beispielhaft genannte Reihenfolge der Umsetzungen verdeutlicht lediglich die bevorzugte Arbeitsweise. Es wäre im Prinzip auch möglich, zur Blockierung des Ausgangspolyisocyanats ein Gemisch aus den Blockierungsmitteln A) und B) einzusetzen. Falls zusätzliche Blockierungsmittel C) zum Einsatz gelangen, können diese ebenfalls zu einem beliebigen Zeitpunkt, gegebenenfalls in Abmischung mit dem Blockierungsmittel A), oder dem Blockierungsmittel B) oder mit dem Gemisch dieser beiden Blockierungsmittel eingesetzt werden. Die vorstehend genannte Reihenfolge der Umsetzung ist jedoch bevorzugt, weil es Ziel dieser Umsetzungen ist, nach der möglichst vollständigen Blockierung der NCO-Gruppen in dem Bindemittel keine überschüssigen Blockierungsmittel vorliegen zu haben. Dem zufolge wird vorzugsweise das weniger reaktive Blockierungsmittel zuerst und das höher reaktive am Schluß mit dem verbleibenden Rest der NCO-Gruppen umgesetzt.

Die erfindungsgemäßen, überwiegend bzw. vollständig blockierten Polyisocyanate stellen wertvolle Vernetzerharze für organische Polyhydroxylverbindungen bei der Herstellung von Einbrennlacken dar. Sie können hierbei anstelle der bislang für diesen Zweck eingesetzten blockierten Polyisocyanate eingesetzt werden. Geeignete Polyhydroxylverbindungen für diesen Einsatzzweck, sowie weitere Details bezüglich der Herstellung und Anwendung derartiger Einbrennlacke können der einschlägigen Literatur entnommen werden. Besonders bevorzugtes Anwendungsgebiet für die erfindungsgemäßen Produkte ist ihre Verwendung als Vernetzer für Einbrenn-Polyurethanklarlacke, wie sie als Decklack insbesondere bei der Automobil-Mehrschichtlackierung zur Anwendung gelangen, wobei als Reaktionspartner für die erfindungsgemäßen blockierten Polyisocyanate die an sich bekannten Polyesterpolyole, Polyacrylatpolyole oder deren Gemische verwendet werden.

In den nachfolgenden Beispielen beziehen sich alle Prozentangaben, soweit nichts anderslautendes angemerkt, auf das Gewicht.

### Beispiel 1 (Vergleichsbeispiel)

Dieses Beispiel beschreibt die Blockierung eines typischen Lackpolyisocyanates auf Basis von 1,6-Diisocyanatohexan mit 1,2,4-Triazol. Es resultiert ein unbrauchbares, da kristallisierendes Produkt.

| Ansatz | | |
|---|---|---|
| 200,0 g | (1,0 Val) | eines isocyanurathaltigen Lackpolyisocyanates auf Basis von 1,6-Diisocyanatohexan. NCO-Gehalt: 21 %, Viskosität bei 23°C ca. 3000 mPas |
| 72,5 g | (1,05 Mol) | 1,2,4-Triazol |
| 117,0 g | | Methoxy-propylacet |
| 389,5 g | (1,0 Val block. NCO-Gruppen) | |

### Durchführung

Das oben genannte, Lösungsmittel-freie Lackpolyisocyanat wird im Methoxypropylacetat gelöst. Bei Raumtemperatur gibt man dann zu der gerührten Lösung die Gesamtmenge des 1,2,4-Triazols und erhitzt allmählich auf 100°C. Hierbei geht das Blockierungsmittel in Lösung. Die Lösung wird während einer weiteren Stunde bei 100°C gerührt. Nach Verschwinden der NCO-Bande (IR-Spektrum) läßt man abkühlen. Während dieses Abkühlens wird die Lösung trüb, nach Lagerung über Nacht tritt vollständige Kristallisation ein.

### Beispiel 2 (Vergleichsbeispiel)

Dieses Beispiel beschreibt die Blockierung eines typischen Lackpolyisocyanates auf Basis von IPDI mit 1,2,4-Triazol. Es resultiert ein blockiertes Lackpolyisocyanat mit einer für high solid Anwendungen zu hohen Viskosität.

| **Ansatz** | | |
|---|---|---|
| 350,0 g | (1,0 Val) | eines isocyanurathaltigen Lackpolyisocyanates auf Basis von IPDI, 70 %ig gelöst in Solventnaphtha 100, NCO-Gehalt: 12 % Viskosität bei 23°C ca. 150 mPas |
| 72,5 g | (1,05 Mol) | 1,2,4-Triazol |
| 65,0 g | | Methoxy-propylacet |
| 487,5 g | (1,0 Val block. NCO-Gruppen) Gehalt an blockierten NCO-Gruppen: Ber. 8,6 % Festkörpergehalt: 65 % | |

### Durchführung

Das obige Lackpolyisocyanat und Methoxypropylacetat werden vorgelegt und gerührt. Hierzu gibt man allmählich das in Form von weißen Schuppen vorliegende 1,2,4-Triazol und erwärmt unter Rühren auf 100 °C. Nach einer Umsetzungsdauer von ca. 6 Stunden ist IR-spektroskopisch fast kein Gehalt an NCO-Gruppen mehr festzustellen. Man läßt erkalten und erhält eine klare, blaß gelb gefärbte Lösung des blockierten Polyisocyanates. Diese 65%ige Lösung weist bei 23°C eine Viskosität von 60 000 mPa.s auf.

### Beispiel 3 (erfindungsgemäß)

Dieses Beispiel beschreibt die erfindungsgemäße Mischblockierung anhand des in Beispiel 1 verwendeten Polyisocyanates auf Basis von 1,6-Diisocyanatohexan. Im Unterschied zu Beispiel 1 entsteht aber hier ein flüssiges, nicht kristallisierendes blockiertes Lackpolyisocyanat.

| **Ansatz** | | |
|---|---|---|
| 400,0 g | (2,0 Val) | eines isocyanurathaltigen Lackpolyisocyanates auf Basis von 1,6-Diisocyanatohexan gemäß Beispiel 1 |
| 69,0 g | (1,0 Mol) | 1,2,4-Triazol |
| 96,0 g | (1,0 Mol) | 3,5-Dimethylpyxrazol |
| 242,0 g | | Methoxy-propylacet |
| 807,0 g | (2,0 Val block. NCO-Gruppen) | |

### Durchführung

Polyisocyanat und Methoxypropylacetat werden vorgelegt. Unter Rühren wird festes 1,2,4-Triazol (weiße Flocken) hinzugegeben. Man erhitzt auf 100 °C, wobei 1,2,4-Triazol in Lösung geht. Nach einer Umsetzungsdauer von 30 min wird ein NCO-Gehalt von 5,5 % gemessen (berechnet 5,9 %). Man kühlt auf 70 °C ab und gibt portionsweise 3,5-Dimethylpyrazol (farblose Kristalle) hinzu. Nach 30 min Umsetzungsdauer bei 70 °C ist IR-spektroskopisch kein NCO-Gehalt mehr nachweisbar. Man erhält eine klare, hellgelbe 70%ige Lösung mit einer Viskosität bei 23 °C von ca. 3000 mPas. Das gelöste, blockierte Polyisocyanat weist einen Gehalt an blockierten NCO-Gruppen (berechnet als NCO und bezogen auf Feststoff) von 14,8 % auf. Die Eigenschaften eines mit diesem blockierten Lackpolyisocyanat hergestellten Klarlackes werden in Beispiel 5 beschrieben.

### Beispiel 4 (erfindungsgemäß)

Dieses Beispiel beschreibt die erfindungsgemäße Mischblockierung des in Beispiel 2 eingesetzten Lackpolyisocyanat auf Basis von IPDI. Im Unterschied zu Beispiel 2 entsteht hier ein relativ niedrig viskoses blockiertes Polyisocyanat.

| **Ansatz** | | |
|---|---|---|
| 700,0 g | (2,0 Val) | eines isocyanurathaltigen Lackpolyisocyanates auf Basis von IPDI gemäß Beispiel 2 |
| 69,0 g | (1,0 Mol) | 1,2,4-Triazol |
| 96,0 g | (1,0 Mol) | 3,5-Dimethylpyrazol |
| 143,0 g | | Methoxy-propylacet |
| 1008,0 g | (2,0 Val block. NCO-Gruppen) | |

### Durchführung

Polyisocyanat und Methoxypropylacetat werden vorgelegt. Unter Rühren wird festes 1,2,4-Triazol (weiße Flocken) hinzugegeben. Man erhitzt auf 100 °C, wobei 1,2,4-Triazol in Lösung geht. Nach ca. 30 min Umsetzungsdauer bei 100 °C wird ein NCO-Gehalt von 4,4 % gemessen, berechnet sind 4,6 %. Man kühlt auf 70 °C ab und gibt 3,5-Dimethylpyrazol (farblose Kristalle) hinzu. Nach 30 min Umsetzungsdauer bei 70 °C ist keine NCO-Gruppe mehr nachweisbar (IR-Spektrum). Man erhält eine klare, blaß-gelbe 65%ige Lösung mit einer Viskosität bei 23 °C von ca. 12000 mPas. Das gelöste, blockierte Polyisocyanat weist ein Gehalt an blockierten NCO-Gruppen (berechnet als NCO und bezogen auf Feststoff) von 12,8 % auf. Die Eigenschaften eines mit diesem blockierten Lackpolyisocyanat hergestellten Klarlackes werden in Beispiel 5 beschrieben.

### Beispiel 5 (erfindungsgemäß)

Es werden Klarlackkombinationen mit den erfindungsgemäßen blockierten Vernetzern aus den Beispielen 3 und 4 sowie die Thermovergilbung beschrieben.

### 1. Klarlack-Aufbau

Für die Herstellung der Klarlacke wird nachfolgende Polyolkomponente, bestehend aus einem "harten" Acrylat und einem "flexibilisierenden" Polyester, eingesetzt:

| | | | |
|---|---|---|---|
| 80 | OH-Äquivalent-% | Acrylat I | 290 g |
| 20 | OH-Äquivalent-% | Polyester I | 90 g |
| 100 | OH-Äquivalen-% | 1 Val OH-Komponente | 380 g |

Bei dem Acrylat I handelt es sich um eine 75 %ige Lösung in Xylol eines handelsüblichen Polyacrylatharzes mit einem Hydroxylgruppengehalt der Lösung von 4,7 % (Desmophen A Versuchsprodukt LS 2051 der Bayer AG, Leverkusen).

Bei dem Polyester I handelt es sich um eine 80 %ige Lösung in n-Butylacetat eines handelsüblichen, verzweigten Polyesterpolyols (Desmophen Versuchsprodukt LS 2971 der Bayer AG, Leverkusen). Der Hydroxylgruppengehalt der Lösung liegt bei 3,8 %.

Die Klarlacke werden durch Mischen der obigen Polyolkomponente (Acrylat/Polyester) mit den erfindungsgemäßen Vernetzern der Beispiele 3 und 4 im Verhältnis ihrer Äquivalent-Gewichte (NCO/OH = 1) unter Zugabe eines Katalysators, wie nachfolgend aufgelistet, hergestellt.

| Klarlackrezeptur | Polyisocyanat | Polyolmischung Acrylat/Polyester | Katalyse 0,1 % Dibutylzinnlaurat |
|---|---|---|---|
| A | Beispiel 3 403 g | 380 g | 0,8 g |
| B | Beispiel 4 504 g | 380 g | 0,9 g |

### 2. Applikation und Thermovergilbung

Die obigen Klarlacke werden auf Prüfbleche, die mit einem handelsüblichen, weißen Basislack (®Permacron Mischlack Serie 293 MB 501 weiß, z. B. von der Fa. Spies & Hecker/Köln), beschichtet sind, in einer Naßschichtdicke von ca. 120 bis 150 µm appliziert.

Die Prüfbleche werden anschließend für 30 Minuten bei 140 °C im Trockenschrank eingebrannt. Danach erfolgt die erste Farbmessung nach der sogenannten CIE-LAB-Methode (DIN 6174). Je größer der hierbei ermittelte positive b-Wert ist, um so mehr hat sich der Klarlack verfärbt.

Hieran schließt sich der Überbrennvorgang mit 30 Minuten bei 160 °C an, anschließend wird der Zuwachs der Gelbfärbung, der sogenannte Δb-Wert nach dem CIE-LAB-Farbsystem (DIN 6174) gemessen. Dieser Wert sollte für überbrennfeste Klarlacke möglichst nahe bei 0 sein.

Die Ergebnisse der Klarlacke A und B sind, wie folgt, zusammengefaßt.

| Klarlack | Thermovergilbung nach dem Einbrennvorgang (b) | Thermovergilbung nach dem Überbrennvorgang (Δb) | Schichtdicke (µm) |
|---|---|---|---|
| A | 1,4 | 1,0 | 55 |
| B | -0,2 | 0,0 | 55 |

Die obige Messung zeigt, daß Klarlackfilm B praktisch keine Vergilbung aufweist.

Im Fall des Lackfilms A) ergibt die Summe der Einzelwerte b) und Δb) den Wert 2,4. Ein analoger Klarlack auf Basis eines analogen Butanonoxim-blockierten Polyisocyanats ergibt unter gleichen Versuchsbedingungen einen Wert b + Δb von 5,1.

## Patentansprüche

1. (Cyclo)aliphatische Polyisocyanate, deren Isocyanatgruppen zu mindestens 95 % in mit Blockierungsmitteln blockierter Form vorliegen, und die einen Gehalt an unblockierten und blockierten Isocyanatgruppen (berechnet als NCO) von insgesamt 5 bis 25 Gew.-% aufweisen, dadurch gekennzeichnet, daß sich die Blockierungsmittel zu
A) 30 bis 70 Äquivalent-% aus 1,2,4-Triazol, zu
B) 30 bis 70 Äquivalent-% aus 3,5-Dimethylpyrazol und zu
C) 0 bis 30 Äquivalent-% aus anderen, von A) und B) verschiedenen Blockierungsmitteln zusammensetzen,
wobei sich die genannten Prozentsätze auf die Gesamtheit der Blockierungsmittel beziehen und zu 100 ergänzen.

2. Verfahren zur Herstellung von (cyclo)aliphatischen Polyisocyanaten gemäß Anspruch 1, gegebenenfalls in in Lacklösungsmitteln gelöster Form durch Umsetzung einer, gegebenenfalls in Lacklösungsmitteln gelösten Polyisocyanat-Komponente, bestehend aus mindestens einem Polyisocyanat mit (cyclo)aliphatisch gebundenen Isocyanatgruppen und einem Isocyanatgehalt von 7 bis 30 Gew.-% mit Blockierungsmitteln für Isocyanatgruppen, wobei gegebenenfalls vor, während und/oder im Anschluß an die Blockierungsreaktion bis zu 20 NCO-Äquivalent-% des zu blockierenden Polyisocyanats mit Verbindungen mit mindestens einer Carbonsäurehydrazid-Gruppe und mindestens einer gegenüber Isocyanatgruppen reaktionsfähigen Gruppe im Sinne einer Isocyanat-Additionsreaktion zur Reaktion gebracht werden, so daß das resultierende, blockierte Polyisocyanat bis zu 5 Gew.-%, bezogen auf Feststoff, an chemisch fixierten Struktureinheiten der Formel 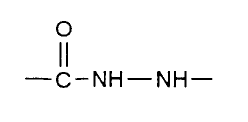 enthält,
dadurch gekennzeichnet, daß die Blockierungsmittel in einer Menge von mindestens 95 Äquivalent-%, bezogen auf die, nach der genannten, gegebenenfalls durchzuführenden Modifizierung noch vorliegenden NCO-Gruppen des zu blockierenden Polyisocyanats zum Einsatz gelangen und sich, bezogen auf die Gesamtmenge der Blockierungsmittel, zu
A) 30 bis 70 Äquivalent-% aus 1,2,4-Triazol, zu
B) 30 bis 70 Äquivalent-% 3,5-Dimethylpyrazol und zu
C) 0 bis 30 Äquivalent-% aus mindestens einem weiteren, von A) und B) verschiedenen Blockierungsmittel zusammensetzen, wobei die Blockierungsmittel in beliebiger Reihenfolge oder als Gemisch zum Einsatz gelangen.

3. Verwendung der (cyclo)aliphatischen Polyisocyanate gemäß Anspruch 1 als Vernetzer für organische Polyhydroxylverbindungen in Polyurethan-Einkomponenten-Einbrennlacken.

## Claims

1. (Cyclo)aliphatic polyisocyanates, at least 95 % of the isocyanate groups of which are present in a form in which they are blocked by means of blocking agents, and which have a total content of blocked and non-blocked isocyanate groups (calculated as NCO) of 5 to 25 weight %, characterized in that the blocking agents are composed of
A) 30 to 70 equivalent % of 1,2,4-triazole,
B) 30 to 70 equivalent % of 3,5-dimethylpyrazole, and
C) 0 to 30 equivalent % of other blocking agents which are different from A) and B),
wherein the said percentages are based on the totality of the blocking agents and add up to 100.

2. A method of preparing (cyclo)aliphatic polyisocyanates according to claim 1, optionally in dissolved form in lacquer solvents, by the reaction of a polyisocyanate component, optionally dissolved in lacquer solvents, consisting of at least one polyisocyanate having (cyclo)aliphatically bonded isocyanate groups and an isocyanate content of 7 to 30 weight %, with blocking agents for isocyanate groups, wherein optionally up to 20 NCO equivalent % of the polyisocyanate to be blocked is reacted before, during and/or after the blocking reaction with compounds having at least one carboxylic acid hydrazide group and at least one group which is capable of reacting with isocyanate groups in the sense of an isocyanate addition reaction, so that the resulting blocked polyisocyanate contains up to 5 weight %, based on the solid, of chemically fixed structural units of formula 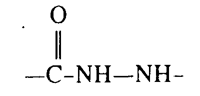 characterized in that the blocking agents are used in an amount of at least 95 equivalent %, based on the NCO groups of the polyisocyanate to be blocked which are still present after the said modification which is optionally to be effected and are composed, based on the total amount of blocking agents, of
A) 30 to 70 equivalent % of 1,2,4-triazole,
B) 30 to 70 equivalent % of 3,5-dimethylpyrazole, and
C) 0 to 30 equivalent % of at least one additional blocking agent which is different from A) and B), wherein the blocking agents are used in any sequence or as a mixture.

3. The use of the (cyclo)aliphatic polyisocyanates according to claim 1 as crosslinking agents for organic polyhydroxyl compounds in single-component polyurethane stoving finishes.

## Revendications

1. Polyisocyanates (cyclo)aliphatiques dont les radicaux isocyanate sont présents à au moins 95% sous forme bloquée avec des agents de blocage, et qui présentent une teneur en radicaux isocyanate non bloqués et bloqués (calculée sous forme de NCO) d'au total 5 à 25% en poids, caractérisés en ce que les agents de blocage se composent de :
A) 30 à 70% en équivalents de 1,2,4-triazole,
B) 30 à 70% en équivalents de 3,5-diméthylpyrazole, et
C) 0 à 30% en équivalents d'autres agents de blocage différents de A) et de B),
où les pourcentages cités se calculent sur la totalité de l'agent de blocage et s'additionnent pour faire 100.

2. Procédé de préparation de polyisocyanates (cyclo)aliphatiques suivant la revendication 1, facultativement sous forme dissoute dans un solvant pour peinture, par réaction d'un composant polyisocyanate facultativement dissous dans un solvant pour peinture, consistant en au moins un polyisocyanate avec des radicaux isocyanate liés à des parties (cyclo)aliphatiques et avec une teneur en isocyanate allant de 7 à 30% en poids, avec des agents de blocage pour les radicaux isocyanate, où facultativement avant, pendant et/ou à la suite de la réaction de blocage, jusqu'à 20% en équivalents des NCO des polyisocyanates à bloquer sont mis à réagir avec des composés ayant au moins un radical hydrazide d'acide carboxylique et au moins un radical capable de réagir avec les radicaux isocyanate, dans le sens d'une réaction d'addition sur isocyanate, de sorte que le polyisocyanate bloqué, résultant, contient jusqu'à 5% en poids, sur base des matières solides, d'unités structurales chimiquement fixées de formule : 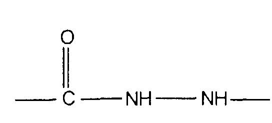 caractérisé en ce que l'agent de blocage est utilisé en une quantité d'au moins 95% en équivalents sur base des radicaux NCO encore présents après la modification citée, facultativement réalisée, du polyisocyanate à bloquer et se compose, sur base de la quantité totale de l'agent de blocage, de :
A) 30 à 70% en équivalents de 1,2,4-triazole;
B) 30 à 70% en équivalents de 3,5-diméthylpyrazole, et
C) 0 à 30 en équivalents d'au moins un autre agent de blocage différent de A) et de B), où les agents de blocage sont utilisés suivant un ordre quelconque ou en mélange.

3. Utilisation de polyisocyanates (cyclo)aliphatiques suivant la revendication 1, comme agents de réticulation pour des composés polyhydroxylés organiques dans des laques émail thermodurcissables à un composant, en polyuréthanne.
